# EUROPEAN PATENT SPECIFICATION

(11) **EP 2 441 477 B1**
(45) Date of publication and mention of the grant of the patent: **13.02.2019**
(21) Application number: 10786242.7
(22) Date of filing: 11.06.2010
(51) Int. Cl.: A61L 15/64, A61K 9/70, A61K 38/43, A61K 38/48, A61K 47/02, A61K 47/12, A61K 47/30, A61K 47/34, A61K 47/42, A61P 17/02, A61K 38/36, A61L 15/44, A61L 15/26, A61L 15/32

(54) **WOUND-COVERING MATERIAL**
WUNDABDECKMATERIAL
MATÉRIAU DE RECOUVREMENT D'UNE PLAIE

(30) Priority: 11.06.2009 JP 2009140456
(43) Date of publication of application: 18.04.2012
(73) Proprietor: KM Biologics Co., Ltd., Kumamoto 860-8568 (JP)
(72) Inventor: ASATO, Ryo, Kyoto-shi Kyoto 612-8555 (JP); SHINYA, Noriko, Kikuchi-shi Kumamoto 869-1298 (JP); ISHIZAWA, Yuki, Kikuchi-shi Kumamoto 869-1298 (JP); HARANO, Satomi, Kikuchi-shi Kumamoto 869-1298 (JP)
(74) Representative: Vossius & Partner Patentanwälte Rechtsanwälte mbB
(86) International application number: PCT/JP2010/059945
(87) International publication number: WO 2010/143711

(56) References cited:
- EP-A1- 1 588 722
- EP-A1- 1 759 718
- EP-A1- 2 130 549
- WO-A1-96/40174
- WO-A1-99/56798
- WO-A1-99/59647
- WO-A1-2004/064878
- WO-A1-2008/117746
- JP-A- 4 272 764
- JP-A- 9 262 249
- JP-A- 11 309 151
- JP-A- 2004 248 984
- JP-A- 2006 306 759
- JP-A- 2009 183 649
- JP-T- 9 504 719
- JP-T- 2000 510 357
- JP-T- 2002 515 300

## Description

### TECHNICAL FIELD OF THE INVENTION

The present invention relates to a wound-covering material which comprises thrombin, fibrinogen and a bioabsorbable supporting material and which may easily be stuck to a deficient area in the skin caused by trauma, surgical operation, burn, and the like through adhering property of its own to thereby protect and repair a wound surface.

### BACKGROUND OF THE INVENTION

When deficiency occurs in the skin after surgical operation, trauma, and the like, material to cover and protect the wound surface is necessary. For a temporary measure, a gauze or an absorbent cotton may be used. However, a gauze or an absorbent cotton is problematic in that the wound surface may be destroyed when they are peeled off to cause bleeding again.

In recent years, various kinds of wound-covering material have been developed. For instance, there have been developed a film made of e.g. polyurethane and a wound-covering material called hydrocolloid dressing (e.g. Patent Reference 1). However, these are ones that do not positively support cure through auto-regeneration in the wound area but in principle is aimed mainly at maintaining moisture circumstances. Therefore, they basically depend on capacity of auto-regeneration and thus promotion of cure at the wound area would not be expected. Besides, due to their highly occlusive property, they may occasionally retain exudates in the suffered area to thereby retard cure.

In case of a full-thickness skin wound in the skin that is in a wide range and to the depth of the dermis, including a wound after surgery of removal of necrotic tissue for treatment of severe burn, cure of spontaneous regeneration would not be expected to necessitate transplantation of said tissue. For this purpose, autotransplantation or allotransplantation of the skin has hitherto been performed. However, in case of autotransplantation, of the skin, deficiency in the skin tissue is newly generated at the area where the skin was removed for transplantation to further necessitate regeneration of the skin at the deficient area. Besides, autotransplantation of the skin has restriction that it requires highly technical treatment in view of plastic surgery, that it is preferably applied to patients in good systemic conditions, and that it cannot be used in emergency. On the other hand, in case of allotransplantation of the skin, the skin is peeled off a week to 10 days after transplantation and thus is merely a temporary covering measure to necessitate transplantation of the skin from the patient.

In recent years, for regeneration of deficient skin, culture of epidermal cells alone to regenerate an epidermal layer (cultured epidermis) or transplantation of collagen sponge to the living body to regenerate dermis-like tissue (artificial dermis) has been performed (e.g. Non-patent References 1 and 2). However, the cultured epidermis may occasionally cause problems such as a low rate of take in case of severely deficient skin to the depth of the dermis as the cultured epidermis does not contain the dermis, and even in case of the take occurring, unevenness remains after closure of the wound, extreme promotion of wound contraction to cause scar contracture, suffering of pain, and the like. In case of the artificial dermis, there is also a problem of a low rate of take since animal-derived collagen is transplanted under the epidermis to thereby induce immune reaction to this substance and high infectivity. Also, some artificial dermis may require laborsome pretreatment such as repetition of immersion and washing in a saline before application.

In order to increase a rate of take, an approach has been developed where cultured dermis is spread over collagen gel or collagen sponge and thereupon epidermal cells are overlaid to construct a skin-like structure, which is transplanted (e.g. Patent Reference 2). However, this approach is also problematic in that, since living cells are used in an artificial skin with cultured cells, laborsome procedures are necessary for isolation of the cells, and transfer and storage of the cells are difficult. Furthermore, it will take time as long as several weeks for culture of the skin. Therefore, at the time when an artificial skin in an amount sufficient for covering the wound surface is obtained, the wound surface especially of burn has sometimes been afflicted wit infection to render transplantation and the take of an artificial skin difficult.

Besides, the currently used wound-covering material and artificial skin basically lack adhesiveness and therefore sometimes may cause a problem when applied depending on a site or shape of a wound. When they are used with a supplementary fixing material such as an adhesive plaster and a film material, space for applying a fixing material becomes necessary to render their use at eyelids or lips inconvenient. When they are used with suture, the current dosage form of sponge with thickness would render their application to the wound surface in a complicated shape difficult. Skin trauma mostly occurs at fingers, limbs and face, all of which have a complicated, steric structure. Therefore, it is a great concern whether the currently used wound-covering material or artificial skin may protect the wound surface in compliance with the shape of the wound surface.
Patent reference 3 discloses compositions comprising hemostatic compounds and bioabsorbable polymers.
Patent reference 1: Japanese patent publication No. 9-262249
Patent reference 2: Japanese patent publication No. 6-292568
Patent reference 3: WO 99/56798

Non-patent reference 1: Ichiro Ono et al., Japan Surgical Society, 100 (9): 522-529, 1999
Non-patent reference 2: Kazuya Matsuda et 1., Burns, 17(2): 29-35, 1991

### DISCLOSURE OF THE INVENTION

### (Technical Problem to be Solved by the Invention)

For dissolving the problems mentioned above, one measure is to develop a wound-covering material that is capable of application as promptly as possible after a wound is produced, has adhesiveness of its own, is easily suited to a deficient area of the skin to be closely adhered thereto through elasticity and flexibility to protect the wound surface, and is capable of reconstructing deficient tissue of the skin in a prompt and good manner while relieving scar contracture and pain.

### (Means for Solving the Problems)

In view of the above-mentioned various problems, the resent inventors have carried out intensive investigation and as a consequence found that a wound-covering material comprising thrombin and fibrinogen as an effective ingredient and a bioabsorbable supporting material as a substrate exerted extremely excellent effects to thereby complete the present invention.

The invention is defined in the claims.

Specifically, the present invention encompasses the following embodiments.
1. A wound-covering material comprising a first layer comprising thrombin and fibrinogen as an effective ingredient and a bioabsorbable supporting material as a substrate, said layer being overlaid with a second layer comprising a covering material for retaining moisture which is made of silicone or polyurethane.
2. The wound-covering material according to item 1, wherein said first layer is consisted of either
   (1) fibrinogen and a bioabsorbable supporting material holding thrombin; or
   (2) a bioabsorbable supporting material holding thrombin, and a bioabsorbable supporting material holding fibrinogen; or
   (3) thrombin, fibrinogen and a bioabsorbable supporting material;
   wherein thrombin and fibrinogen are separated from each other immediately prior to use thereof.
3. The wound-covering material according to item 1 or 2, wherein said bioabsorbable supporting material is made of a material selected from the group consisting of polyglycolic acid, polylactic acid and a copolymer of glycolic acid and lactic acid.
4. The wound-covering material according to any one of items 1 to 3, wherein said bioabsorbable supporting material is processed to a non-woven fabric.
5. The wound-covering material according to any one of items 1 to 4, wherein said bioabsorbable supporting material is a non-woven fabric made of a material of polyglycolic acid.
6. The wound-covering material according to any one of items 1 to 5, wherein said wound-covering material comprises at least one additive selected from Blood Coagulation Factor XIII, a protease inhibitor, calcium chloride, albumin, sodium chloride, sodium citrate, or a non-ionic detergent.
7. The wound-covering material according to any one of items 1 to 6, wherein said wound-covering material may repair a deficient area in the skin due to trauma, surgical operation or burn.
8. A kit for the wound-covering material as defined in claim 1, said kit comprising a bioabsorbable supporting material holding thrombin as an effective ingredient, a container containing fibrinogen as an effective ingredient, and a covering material for retaining moisture which is made of silicone or polyurethane.
9. The wound-covering material kit according to item 8, wherein said bioabsorbable supporting material is made of a material selected from the group consisting of polyglycolic acid, polylactic acid and a copolymer of glycolic acid and lactic acid.
10. The wound-covering material kit according to item 8 or 9, wherein said bioabsorbable supporting material is processed to a non-woven fabric.
11. The wound-covering material kit according to any one of items 8 to 10, wherein said bioabsorbable supporting material is a non-woven fabric made of a material of polyglycolic acid.
12. The wound-covering material kit according to any one of items 8 to 11, wherein said wound-covering material comprises at least one additive selected from Blood Coagulation Factor XIII, a protease inhibitor, calcium chloride, albumin, sodium chloride, sodium citrate, or a non-ionic detergent.
13. The wound-covering material kit according to item 12, wherein said calcium chloride is added to the bioabsorbable supporting material holding thrombin as an additive for thrombin.
14. The wound-covering material kit according to item 12, wherein said Blood Coagulation Factor XIII is contained in the container containing fibrinogen.
15. The wound-covering material kit according to any one of items 8 to 14, wherein said bioabsorbable supporting material is prepared by a process comprising the step of immersing a bioabsorbable supporting material in a solution containing thrombin, and the step of lyophilizing the supporting material obtained by said step.
16. The wound-covering material kit according to any one of items 8 to 15, wherein said wound-covering material may repair a deficient area in the skin due to trauma, surgical operation or burn.
17. A kit for the wound-covering material as defined in claim 1, said kit comprising a bioabsorbable supporting material holding thrombin as an effective ingredient, a bioabsorbable supporting material holding fibrinogen as an effective ingredient, and a covering material for retaining moisture which is made of silicone or polyurethane.
18. The wound-covering material kit according to item 17, wherein said bioabsorbable supporting material is made of a material selected from the group consisting of polyglycolic acid, polylactic acid and a copolymer of glycolic acid and lactic acid.
19. The wound-covering material kit according to item 17 or 18, wherein said bioabsorbable supporting material is processed to a non-woven fabric.
20. The wound-covering material kit according to any one of items 17 to 19, wherein said bioabsorbable supporting material is a non-woven fabric made of a material of polyglycolic acid.
21. The wound-covering material kit according to any one of items 17 to 20, wherein said wound-covering material comprises at least one additive selected from Blood Coagulation Factor XIII, a protease inhibitor, calcium chloride, albumin, sodium chloride, sodium citrate, or a non-ionic detergent.
22. The wound-covering material kit according to item 21, wherein said calcium chloride is added to the bioabsorbable supporting material holding thrombin as an additive for thrombin.
23. The wound-covering material kit according to item 21, wherein said non-ionic detergent is contained in the bioabsorbable supporting material holding fibrinogen.
24. The wound-covering material kit according to any one of items 17 to 23, wherein said bioabsorbable supporting material holding thrombin or said bioabsorbable supporting material holding fibrinogen is prepared by a process comprising the step of immersing a bioabsorbable supporting material in a solution containing thrombin or fibrinogen, and the step of lyophilizing the supporting material obtained by said step.
25. The wound-covering material kit according to any one of items 17 to 24, wherein said wound-covering material may repair a deficient area in the skin due to trauma, surgical operation or burn.
26. A kit for the wound-covering material as defined in claim 1, said kit comprising a containing thrombin as an effective ingredient, a container containing fibrinogen as an effective ingredient, a bioabsorbable supporting material, and a covering material for retaining moisture which is made of silicone or polyurethane.
27. The wound-covering material kit according to item 26, wherein said bioabsorbable supporting material is made of a material selected from the group consisting of polyglycolic acid, polylactic acid and a copolymer of glycolic acid and lactic acid.
28. The wound-covering material kit according to item 26 or 27, wherein said bioabsorbable supporting material is processed to a non-woven fabric.
29. The wound-covering material kit according to any one of items 26 to 28, wherein said bioabsorbable supporting material is a non-woven fabric made of a material of polyglycolic acid.
30. The wound-covering material kit according to any one of items 26 to 29, wherein said wound-covering material comprises at least one additive selected from Blood Coagulation Factor XIII, a protease inhibitor, calcium chloride, albumin, sodium chloride, sodium citrate, or a non-ionic detergent.
31. The wound-covering material kit according to item 30 wherein said calcium chloride is added to the container containing thrombin as an additive for thrombin.
32. The wound-covering material kit according to item 30, wherein said Blood Coagulation Factor XIII is contained in the container containing fibrinogen.
33. The wound-covering material kit according to any one of items 26 to 32, wherein said wound-covering material may repair a deficient area in the skin due to trauma, surgical operation or burn.

### (Effects of the Invention)

It was revealed that a wound-covering material according to the present invention has the following properties and hence may be an ideal wound-covering material.
- It may be applied as promptly as possible after a wound is produced since culture and pretreatment (repetition of immersion and washing with a saline) are not necessary;
- It may be applied through adhesiveness of its own due to fibrin formation and hence suture may be unnecessary;
- With elasticity and flexibility, it may be closely attached to a wound surface in a complicated shape;
- As a result of an adhering and sealing effect, storage of exudates may be inhibited and pain may be alleviated;
- It may advantageously be used for fresh trauma accompanied by bleeding since it may inhibit bleeding through sealing effect;
- Through the activity of thrombin to promote cell growth, it may promote reconstruction of deficient tissue including the epidermis together with formation of granulation tissue;
- After application to a wound surface, it may secure space where granulation tissue is formed with an appropriate strength, may inhibit wound contraction and may decrease scar contracture;
- In case that a covering material for retaining moisture is not overlaid, it may serve as a wound-covering material as protecting a wound surface and being naturally peeled off after cure; in case that a covering material for retaining moisture is optionally overlaid to provide moisture circumstances, it may serve as an artificial skin wherein the rest other than a covering material for retaining moisture serves as a scaffold for cell growth to be replaced with auto-tissue; and
- It is excellent in safety.

### BRIEF DESCRIPTION OF DRAWINGS

Fig. 1 is a photograph of histopathology on Day 10 after application of the wound-covering material of the present invention.
Fig. 2 is a photograph of histopathology, after application of the wound-covering material of the present invention, on Week 3 after being brought to moisture circumstances by covering a covering material for retaining moisture.

### BEST MODE FOR CARRYING OUT THE INVENTION

The present invention relates to a wound-covering material comprising thrombin and fibrinogen as an effective ingredient and a bioabsorbable supporting material as a substrate. The wound-covering material comprises covering material for retaining moisture.

The bioabsorbable supporting material for use in the present invention may be any bioabsorbable synthetic fiber. A bioabsorbable synthetic fiber as used herein refers to a synthetic fiber that is unlikely to induce inflammation in the living body as foreign substance and may be absorbed and/or degraded within the living body with time. The bioabsorbable supporting material has preferably appropriate flexibility and elasticity to ensure that it may surely cover any deficient area in any shape. The bioabsorbable supporting material may preferably be processed into a non-woven fabric. The non-woven fabric may be prepared e.g. by making bioabsorbable material into woven or knitted fabric and then needle-punched to give a non-woven fabric in accordance with the method described in Japanese Patent Publication No. 18579/1993. For example, a synthetic fiber that may form such a non-woven fabric includes polyglycolic acid, polylactic acid, or a copolymer of glycolic acid with lactic acid, etc., which may be used after processing into a non-woven fabric. Among these, a bioabsorbable synthetic non-woven fabric which is prepared from polyglycolic acid by processing into a non-woven fabric is the most preferable material for the purpose of the present invention.

The bioabsorbable supporting material may be in any shape but preferably in the form of a sheet in view of versatility to deficient area of the skin.

In addition to thrombin and fibrinogen as the effective ingredients, a pharmaceutically acceptable stabilizer and additive may also be added. Examples of such stabilizer and additive include, for instance, Blood Coagulation Factor XIII, a protease inhibitor, calcium chloride, albumin, sodium chloride, sodium citrate, a non-ionic detergent, mannitol, and the like.

Thrombin, fibrinogen and Blood Coagulation Factor XIII may preferably be derived from human blood or obtained by the genetic engineering. The wound-covering material of the present invention may be in any dosage form so far as thrombin and fibrinogen as an effective ingredient are ultimately contained in a bioabsorbable supporting material. In view of easy handling under operative settings, however, a bioabsorbable supporting material previously holding thrombin and/or fibrinogen, which maintains flexibility, is one of preferable embodiments from the viewpoint of its easy handling as well as tissue sealing efficacy.

In case that a bioabsorbable supporting material previously holds both thrombin and fibrinogen, the bioabsorbable supporting material should hold each of thrombin and fibrinogen under such condition that the components are separated from each other or each of the components in the form of powder are suspended in an organic solvent and each suspension is sprayed to the non-woven fabric, so that both thrombin and fibrinogen may not react to each other to generate stabilized fibrin.

The kit of the present invention may comprise either:
(A) a bioabsorbable supporting material holding thrombin plus fibrinogen; or
(B) a bioabsorbable supporting material holding thrombin and a bioabsorbable supporting material holding fibrinogen; or
(C) a bioabsorbable supporting material, thrombin, and fibrinogen;
in which a stabilizer and an additive as described above may optionally be added to either of (A) to (C). Namely, the kit of the present invention may comprise embodiments where a bioabsorbable supporting material holds thrombin alone (A), where a bioabsorbable supporting material holds both thrombin and fibrinogen (B), and where a bioabsorbable supporting material holds neither thrombin nor fibrinogen (C) .

For use in case of (A), after fibrinogen is applied to a wound surface, a bioabsorbable supporting material holding thrombin (hereinafter referred to as "supporting material holding thrombin") is overlaid, or alternatively, fibrinogen is applied to a supporting material holding thrombin by spraying or by immersing. Said supporting material holding thrombin may be prepared by (1) dissolving thrombin in a saline or a buffer and optionally adding to the resulting thrombin solution calcium chloride as an additive as appropriate, and (2) immersing the supporting material into said thrombin solution, followed by freezing at -80°C for 2 hours and lyophilization. An amount of thrombin to be held on a supporting material holding thrombin may preferably be 20-100 U/cm².

For use in case of (B), a bioabsorbable supporting material holding fibrinogen (hereinafter referred to as "supporting material holding fibrinogen") and a supporting material holding thrombin are overlaid to each other, and to the supporting material holding fibrinogen put upside is added dropwise distilled water, the surface of which supporting material holding fibrinogen is applied to an afflicted area. Said supporting material holding fibrinogen may be prepared by (1) dissolving fibrinogen in a saline or a buffer and optionally adding to the resulting fibrinogen solution a non-ionic detergent as an additive as appropriate, and (2) immersing the supporting material into said fibrinogen solution, followed by freezing at -80°C for 2 hours and lyophilization. An amount of fibrinogen to be held on a supporting material holding fibrinogen may be 0.5-5 mg/cm², a range of concentration where fibrinogen may exert a hemostatic activity, and preferably 1-4 mg/cm².

For use in case of (C), after fibrinogen, prepared as in the process for preparing a commercially available fibrin sealant (e.g. Bolheal manufactured by Juridical Foundation The Chemo-Sero-Therapeutic Research Institute), is applied to a wound surface, a bioabsorbable supporting material immersed into the solution of thrombin is applied, or alternatively, each of the solutions of thrombin and of fibrinogen is applied simultaneously to the supporting material via spray.

In either case of (A) to (C), Blood Coagulation Factor XIII or a protease inhibitor may be added to a solution containing fibrinogen.

The wound-covering material of the present invention, when applied to a wound surface, may adhere to and protect the wound surface and may be naturally peeled off after cure. In case that an afflicted area is under moisture condition, the wound-covering material may serve as an artificial skin as serving as a scaffold for cell growth to be replaced with auto-tissue.

In case that a wound reaches to the depth of the skin or is spread in a wide range, or in case that an afflicted area is not under moisture condition, a covering material for retaining moisture is used
which is overlaid to the wound-covering material as above. As a consequence, a wound surface may be kept under moisture condition so that the rest other than the covering material for retaining moisture may serve as a scaffold for cell growth to be replaced with auto-tissue. The covering material for retaining moisture as used herein is silicone or polyurethane, preferably a silicone membrane or a urethane membrane.

The wound-covering material obtained in accordance with the present invention, due to its high adhesiveness, appropriate strength, flexibility and elasticity, may be stuck to a deficient area of the skin in any shape to alleviate scar contracture and pain. Besides, since every material used therein is safe to the living body, it may be used in clinical without care.

The present invention is explained in more detail by means of the following Examples but should not be construed to be limited thereto.

### EXAMPLE

### Preparation Example 1: Preparation of supporting material holding thrombin

Thrombin contained in a commercially available tissue sealant kit, Bolheal, Juridical Foundation The Chemo-Sero-Therapeutic Research Institute, was dissolved in a dissolving solution attached to the kit to prepare a thrombin solution at 1875 U/mL of thrombin. Using this thrombin solution at 1875 U/mL of thrombin, a thrombin solution (pH 6.0) was prepared containing 1% glycerol, 3% trehalose, 0.18 M histidine, 40 mM calcium chloride and 0.1% Tween 80, at a final concentration. The thrombin solution (2.5 mL) was soaked evenly into a bioabsorbable synthetic non-woven fabric made of polyglycolic acid (Neoveil, Gunze Limited, 5 x 10 cm, thickness 0.15 mm). This sample, after being frozen and lyophilized for 24 hours, was used as a sample of a supporting material holding thrombin (thrombin held at 93.8 U/cm²).

### Preparation Example 2: Preparation of supporting material holding fibrinogen

Using fibrinogen contained in a commercially available tissue sealant kit, Bolheal, Juridical Foundation The Chemo-Sero-Therapeutic Research Institute, a 8% fibrinogen solution was prepared containing Tween 80 (0.1%) as a non-ionic detergent, albumin (1.0%), NaCl (1.75%), citric acid 3Na (1.2%), glycine (1.5%), and D-mannitol (4.0%). Each of the fibrinogen solution (2.5 mL) was soaked evenly into a bioabsorbable synthetic non-woven fabric made of polyglycolic acid (Neoveil, Gunze Limited, 5 x 10 cm, thickness 0.15 mm). This sample, after being frozen and lyophilized for 24 hours, was used as a sample of a supporting material holding fibrinogen (fibrinogen held at 4 mg/cm²).

### Example 1: Test estimating effect to inhibit scar contracture in guinea pig deficient skin model

### (1) Method of experiment

Induction of anesthesia was performed to guinea pig (male, Hartley, 5 weeks old, Japan SLC, Inc.) with ketamine (DAIICHI SANKYO COMPANY, LIMITED), diazepam (Takeda Pharmaceutical Company Limited) and anesthesia continued by inhalation of isoflurane (Merck) through a mask. At the skin of the back, several full-thickness skin wounds of 1.5 cm square were generated and treated as indicated in each of Groups hereinbelow. For groups 1 and 3, time required for application was measured. On Day 5, Day 8, Day 10, Week 2 and Week 3 after the treatment, photographs of the wound sites were taken and an area of the wounds was obtained by image analysis. Also, on Day 5, Day 10, Week 2, Week 3 and Week 6 after the treatment, each four animals were sacrificed by euthanasia and the treated sites were sampled for histopathology.

### (2) Groups

Group 1: Wound-covering material
After a fibrinogen solution (Bolheal (registered trade mark), Juridical Foundation The Chemo-Sero-Therapeutic Research Institute) was rubbed into the wound sites, a bioabsorbable synthetic non-woven fabric (Neoveil (registered trade mark), Gunze Limited) was applied thereto, onto which the fibrinogen solution and a thrombin solution (Bolheal (registered trade mark), Juridical Foundation The Chemo-Serc-Therapeutic Research Institute) were sprayed.

Group 2: No treatment (negative control): No treatment was carried out to let it be an open wound.

Group 3: Terudermis: Terudermis (registered trade mark; Terumo), a graft for deficiency of the dermis, was applied to the wound sites and sutured with Nylon thread by single-knot suture.

### (3) Results

### (i) Time for application

Time necessary for application was 368.0 ± 27.2 seconds (mean: n=8) for Terudermis whereas it was 44.2±6.1 seconds (mean: n=9) for the wound-covering material. Since the wound-covering material had adhesiveness of its own with no necessity of suture, it could be applied in shorter time, about 1/9, than Terudermis.

### (ii) Area of wound surface

A retention rate of an area of wound was 95.5± 5.75% (n=6) for the wound-covering material whereas it was 73.6±16.21% (n=5) for no treatment and 73.1±18. 02% (n=5) for Terudermis. As such, contraction of wound was inhibited for the wound-covering material as compared to no treatment and Terudermis, on Day 8 after treatment.

### (iii) Histopathological examination

### <Comparison between Groups>

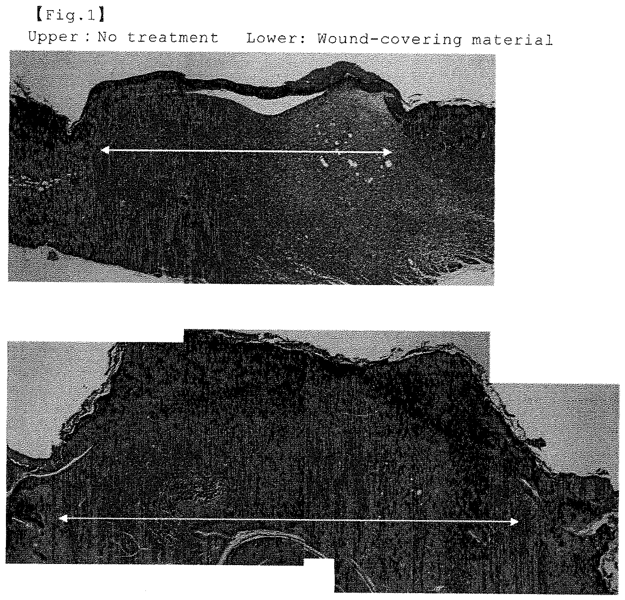

For the wound-covering material, edema and retention of blood and exudates under the crust on Day 5 were lighter than those of no treatment and Terudermis. On Day 10, a depressed area after wound was filled with granulation tissue for every Groups but a distance between the original collagen layers was the most kept for the wound-covering material as compared to no treatment and Terudermis (Fig. 1).

The difference observed on Day 5 may be attributed to the sealing effect of the wound-covering material of the present invention which inhibited retention of blood and exudates. Since retained substance may cause pain or retarding in repair, decrease in these would be expected.

The difference observed on Day 10 may be attributed to appropriate strength of the wound-covering material of the present invention after application which ensures space where granulation tissue is formed and inhibits wound contraction to thereby keep a distance between the original collagen layers. Thus, decrease in scar contracture and pain would be expected by the use of the wound-covering material.

### <Cure by wound-covering material with passage of time>

Day 5: Under the wound-covering material was there a thin layer of exudates, under which fibrosis and regeneration of the epithelium (in portion) from the peripheral region was observed.

Day 10: The wound-covering material was peeled off. The epithelium was regenerated from the peripheral region and under the epithelium neovascularization and formation of granulation tissue were observed.

Week 2: Regeneration of the epithelium was prominent and in some cases complete coverage was observed.

Week 3: Complete coverage by the epithelium was observed. Collagen was seen in the granulation tissue and repair of the dermis and subcutaneous tissue progressed.

Week 6: Continuity of collagen in the dermis was almost recovered. Also, the skin appendage was almost repaired.

As such, no excessive inflammation etc. was observed in the passage of cure with the wound-covering material of the invention and the layers of the epithelium and the dermis were promptly reconstructed.

### Example 2: Observation of histopathology during passage of replacement with auto-tissue under moisture condition: guinea pig deficient skin model

### (1) Method of experiment

Introductory anesthesia was performed to guinea pig (male, Hartley, 5 weeks old, Japan SLC, Inc.) with ketamine (DAIICHI SANKYO COMPANY, LIMITED), diazepam (Takeda Pharmaceutical Company Limited) and anesthesia continued by inhalation of isoflurane (Merck) through a mask. At the skin of the back, several full-thickness skin wounds of 1.5 cm square were generated and treated as indicated in each of Groups hereinbelow. On Day 8, Week 2 and Week 3 after the treatment, the animals were sacrificed by euthanasia and the treated sites were sampled for histopathology.

### (2) Group

Group 1: Wound-covering material: After a fibrinogen solution (Bolheal (registered trade mark), Juridical Foundation The Chemo-Sero-Therapeutic Research Institute) was rubbed into the wound sites, a bioabsorbable synthetic non-woven fabric (Neoveil (registered trade mark), Gunze Limited) was applied thereto, onto which the fibrinogen solution and a thrombin solution (Bolheal (registered trade mark), Juridical Foundation The Chemo-Sero-Therapeutic Research Institute) were sprayed, which was further covered with a covering material for retaining moisture.

### (3) Histopathological examination

Day 8: A depressed area after wound was filled with granulation tissue and regeneration of the epithelium from the peripheral region was observed.

Week 2: Regeneration of the epithelium was prominent and in some cases complete coverage was observed.

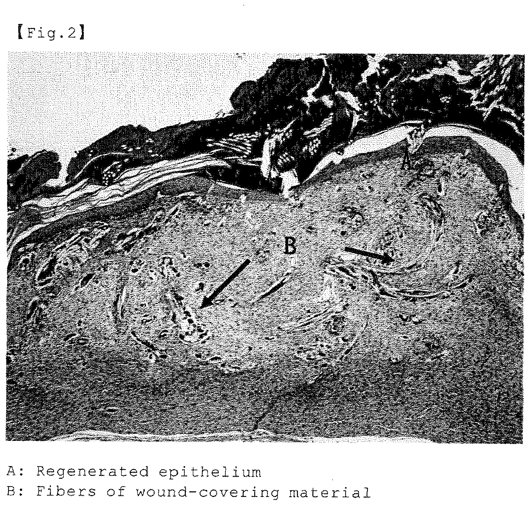

Week 3: With the bioabsorbable non-woven fabric of the wound-covering material and fibrin clot serving as a scaffold, hyperplasia of the connective tissue and neovascularization were observed and the wound-covering material was being replaced with auto-tissue (Fig. 2). It was revealed that, under moisture condition, the wound-covering material was not naturally peeled off after cure of the wound but was replaced with auto-tissue.

### INDUSTRIAL APPLICABILITY

The wound-covering material of the present invention, via its own adhesiveness, may easily be stuck to a deficient area of the skin to protect the wound surface. Besides, the wound-covering material of the present invention, as alleviating scar contracture and pain, may reconstruct deficient tissue of the skin in a prompt and good manner through its biocompatibility and positive repair of tissue. Therefore, the wound-covering material of the present invention may be applied as a wound-covering material or an artificial skin for a deficient area of the skin caused by trauma, surgical operation or burn.

## Claims

1. A wound-covering material comprising a first layer comprising thrombin and fibrinogen as an effective ingredient and a bioabsorbable supporting material as a substrate, said layer being overlaid with a second layer comprising a covering material for retaining moisture which is made of silicone or polyurethane.

2. The wound-covering material according to claim 1, wherein said first layer is consisted of either
(1) fibrinogen and a bioabsorbable supporting material holding thrombin; or
(2) a bioabsorbable supporting material holding thrombin, and a bioabsorbable supporting material holding fibrinogen; or
(3) thrombin, fibrinogen and a bioabsorbable supporting material;
wherein thrombin and fibrinogen are separated from each other immediately prior to use thereof.

3. The wound-covering material according to claim 1 or 2, wherein said bioabsorbable supporting material is made of a material selected from the group consisting of polyglycolic acid, polylactic acid and a copolymer of glycolic acid and lactic acid.

4. The wound-covering material according to any one of claims 1 to 3, wherein said bioabsorbable supporting material is processed to a non-woven fabric.

5. The wound-covering material according to any one of claims 1 to 4, wherein said bioabsorbable supporting material is a non-woven fabric made of a material of polyglycolic acid.

6. The wound-covering material according to any one of claims 1 to 5, wherein said wound-covering material comprises at least one additive selected from Blood Coagulation Factor XIII, a protease inhibitor, calcium chloride, albumin, sodium chloride, sodium citrate, or a non-ionic detergent.

7. The wound-covering material according to any one of claims 1 to 6, wherein said wound-covering material may repair a deficient area in the skin due to trauma, surgical operation or burn.

8. A kit for the wound-covering material as defined in claim 1, said kit comprising a bioabsorbable supporting material holding thrombin as an effective ingredient, a container containing fibrinogen as an effective ingredient, and a covering material for retaining moisture which is made of silicone or polyurethane.

9. The wound-covering material kit according to claim 8, wherein said bioabsorbable supporting material is made of a material selected from the group consisting of polyglycolic acid, polylactic acid and a copolymer of glycolic acid and lactic acid.

10. The wound-covering material kit according to claim 8 or 9, wherein said bioabsorbable supporting material is processed to a non-woven fabric.

11. The wound-covering material kit according to any one of claims 8 to 10, wherein said bioabsorbable supporting material is a non-woven fabric made of a material of polyglycolic acid.

12. The wound-covering material kit according to any one of claims 8 to 11, wherein said wound-covering material comprises at least one additive selected from Blood Coagulation Factor XIII, a protease inhibitor, calcium chloride, albumin, sodium chloride, sodium citrate, or a non-ionic detergent.

13. The wound-covering material kit according to claim 12, wherein said calcium chloride is added to the bioabsorbable supporting material holding thrombin as an additive for thrombin.

14. The wound-covering material kit according to claim 12, wherein said Blood Coagulation Factor XIII is contained in the container containing fibrinogen.

15. The wound-covering material kit according to any one of claims 8 to 14, wherein said bioabsorbable supporting material is prepared by a process comprising the step of immersing a bioabsorbable supporting material in a solution containing thrombin, and the step of lyophilizing the supporting material obtained by said step.

16. The wound-covering material kit according to any one of claims 8 to 15, wherein said wound-covering material may repair a deficient area in the skin due to trauma, surgical operation or burn.

17. A kit for the wound-covering material as defined in claim 1, said kit comprising a bioabsorbable supporting material holding thrombin as an effective ingredient, a bioabsorbable supporting material holding fibrinogen as an effective ingredient, and a covering material for retaining moisture which is made of silicone or polyurethane.

18. The wound-covering material kit according to claim 17, wherein said bioabsorbable supporting material is made of a material selected from the group consisting of polyglycolic acid, polylactic acid and a copolymer of glycolic acid and lactic acid.

19. The wound-covering material kit according to claim 17 or 18, wherein said bioabsorbable supporting material is processed to a non-woven fabric.

20. The wound-covering material kit according to any one of claims 17 to 19, wherein said bioabsorbable supporting material is a non-woven fabric made of a material of polyglycolic acid.

21. The wound-covering material kit according to any one of claims 17 to 20, wherein said wound-covering material comprises at least one additive selected from Blood Coagulation Factor XIII, a protease inhibitor, calcium chloride, albumin, sodium chloride, sodium citrate, or a non-ionic detergent.

22. The wound-covering material kit according to claim 21, wherein said calcium chloride is added to the bioabsorbable supporting material holding thrombin as an additive for thrombin.

23. The wound-covering material kit according to claim 21, wherein said non-ionic detergent is contained in the bioabsorbable supporting material holding fibrinogen.

24. The wound-covering material kit according to any one of claims 17 to 23, wherein said bioabsorbable supporting material holding thrombin or said bioabsorbable supporting material holding fibrinogen is prepared by a process comprising the step of immersing a bioabsorbable supporting material in a solution containing thrombin or fibrinogen, and the step of lyophilizing the supporting material obtained by said step.

25. The wound-covering material kit according to any one of claims 17 to 24, wherein said wound-covering material may repair a deficient area in the skin due to trauma, surgical operation or burn.

26. A kit for the wound-covering material as defined in claim 1, said kit comprising a container containing thrombin as an effective ingredient, a container containing fibrinogen as an effective ingredient, a bioabsorbable supporting material, and a covering material for retaining moisture which is made of silicone or polyurethane.

27. The wound-covering material kit according to claim 26, wherein said bioabsorbable supporting material is made of a material selected from the group consisting of polyglycolic acid, polylactic acid and a copolymer of glycolic acid and lactic acid.

28. The wound-covering material kit according to claim 26 or 27, wherein said bioabsorbable supporting material is processed to a non-woven fabric.

29. The wound-covering material kit according to any one of claims 26 to 28, wherein said bioabsorbable supporting material is a non-woven fabric made of a material of polyglycolic acid.

30. The wound-covering material kit according to any one of claims 26 to 29, wherein said wound-covering material comprises at least one additive selected from Blood Coagulation Factor XIII, a protease inhibitor, calcium chloride, albumin, sodium chloride, sodium citrate, or a non-ionic detergent.

31. The wound-covering material kit according to claim 30, wherein said calcium chloride is added to the container containing thrombin as an additive for thrombin.

32. The wound-covering material kit according to claim 30, wherein said Blood Coagulation Factor XIII is contained in the container containing fibrinogen.

33. The wound-covering material kit according to any one of claims 26 to 32, wherein said wound-covering material may repair a deficient area in the skin due to trauma, surgical operation or burn.

## Patentansprüche

1. Ein Wundabdeckmaterial, umfassend eine erste Schicht, die Thrombin und Fibrinogen als einen wirksamen Bestandteil und ein bioabsorbierbares Trägermaterial als ein Substrat umfasst, wobei die Schicht mit einer zweiten Schicht bedeckt ist, die ein Abdeckmaterial zum Rückhalt von Feuchtigkeit umfasst, das aus Silikon oder Polyurethan besteht.

2. Das Wundabdeckmaterial nach Anspruch 1, wobei die erste Schicht aus entweder
(1) Fibrinogen und einem bioabsorbierbaren Trägermaterial, das Thrombin enthält; oder
(2) einem bioabsorbierbaren Trägermaterial, das Thrombin enthält, und einem bioabsorbierbaren Trägermaterial, das Fibrinogen enthält; oder
(3) Thrombin, Fibrinogen und einem bioabsorbierbaren Trägermaterial besteht; wobei Thrombin und Fibrinogen unmittelbar vor deren Verwendung voneinander getrennt werden.

3. Das Wundabdeckmaterial nach Anspruch 1 oder 2, wobei das bioabsorbierbare Trägermaterial aus einem Material, ausgewählt aus der Gruppe bestehend aus Polyglycolsäure, Polymilchsäure und einem Copolymer von Glycolsäure und Milchsäure, besteht.

4. Das Wundabdeckmaterial nach einem der Ansprüche 1 bis 3, wobei das bioabsorbierbare Trägermaterial zu einem Vlies verarbeitet ist.

5. Das Wundabdeckmaterial nach einem der Ansprüche 1 bis 4, wobei das bioabsorbierbare Trägermaterial ein Vlies aus einem Polyglycolsäure-Material ist.

6. Das Wundabdeckmaterial nach einem der Ansprüche 1 bis 5, wobei das Wundabdeckmaterial mindestens ein Additiv, ausgewählt aus Blutgerinnungsfaktor XIII, einem Proteaseinhibitor, Calciumchlorid, Albumin, Natriumchlorid, Natriumcitrat oder einem nicht-ionischen Detergens, umfasst.

7. Das Wundabdeckmaterial nach einem der Ansprüche 1 bis 6, wobei das Wundabdeckmaterial eine schadhafte Stelle in der Haut aufgrund einer Verletzung, eines chirurgischen Eingriffs oder einer Verbrennung reparieren kann.

8. Ein Kit für das Wundabdeckmaterial wie in Anspruch 1 definiert, wobei das Kit ein bioabsorbierbares Trägermaterial, das Thrombin als einen wirksamen Bestandteil enthält, einen Behälter, der Fibrinogen als einen wirksamen Bestandteil enthält, und ein Abdeckmaterial zum Rückhalt von Feuchtigkeit, das aus Silikon oder Polyurethan besteht, umfasst.

9. Das Wundabdeckmaterial-Kit nach Anspruch 8, wobei das bioabsorbierbare Trägermaterial aus einem Material, ausgewählt aus der Gruppe bestehend aus Polyglycolsäure, Polymilchsäure und einem Copolymer von Glycolsäure und Milchsäure, besteht.

10. Das Wundabdeckmaterial-Kit nach Anspruch 8 oder 9, wobei das bioabsorbierbare Trägermaterial zu einem Vlies verarbeitet ist.

11. Das Wundabdeckmaterial-Kit nach einem der Ansprüche 8 bis 10, wobei das bioabsorbierbare Trägermaterial ein Vlies aus einem Polyglycolsäure-Material ist.

12. Das Wundabdeckmaterial-Kit nach einem der Ansprüche 8 bis 11, wobei das Wundabdeckmaterial mindestens ein Additiv, ausgewählt aus Blutgerinnungsfaktor XIII, einem Proteaseinhibitor, Calciumchlorid, Albumin, Natriumchlorid, Natriumcitrat oder einem nicht-ionischen Detergens, umfasst.

13. Das Wundabdeckmaterial-Kit nach Anspruch 12, wobei das Calciumchlorid dem bioabsorbierbaren Trägermaterial, das Thrombin enthält, als ein Additiv für Thrombin zugegeben ist.

14. Das Wundabdeckmaterial-Kit nach Anspruch 12, wobei der Blutgerinnungsfaktor XIII in dem Fibrinogen enthaltenden Behälter enthalten ist.

15. Das Wundabdeckmaterial-Kit nach einem der Ansprüche 8 bis 14, wobei das bioabsorbierbare Trägermaterial mit Hilfe eines Verfahrens hergestellt ist, das den Schritt des Eintauchens eines bioabsorbierbaren Trägermaterials in eine Thrombin enthaltende Lösung und den Schritt des Lyophilisierens des durch diesen Schritt erhaltenen Trägermaterials umfasst.

16. Das Wundabdeckmaterial-Kit nach einem der Ansprüche 8 bis 15, wobei das Wundabdeckmaterial eine schadhafte Stelle in der Haut aufgrund einer Verletzung, eines chirurgischen Eingriffs oder einer Verbrennung reparieren kann.

17. Ein Kit für das Wundabdeckmaterial wie in Anspruch 1 definiert, wobei das Kit ein bioabsorbierbares Trägermaterial, das Thrombin als einen wirksamen Bestandteil enthält, ein bioabsorbierbares Trägermaterial, das Fibrinogen als einen wirksamen Bestandteil enthält, und ein Abdeckmaterial zum Rückhalt von Feuchtigkeit, das aus Silikon oder Polyurethan besteht, umfasst.

18. Das Wundabdeckmaterial-Kit nach Anspruch 17, wobei das bioabsorbierbare Trägermaterial aus einem Material, ausgewählt aus der Gruppe bestehend aus Polyglycolsäure, Polymilchsäure und einem Copolymer von Glycolsäure und Milchsäure, besteht.

19. Das Wundabdeckmaterial-Kit nach Anspruch 17 oder 18, wobei das bioabsorbierbare Trägermaterial zu einem Vlies verarbeitet ist.

20. Das Wundabdeckmaterial-Kit nach einem der Ansprüche 17 bis 19, wobei das bioabsorbierbare Trägermaterial ein Vlies aus einem Polyglycolsäure-Material ist.

21. Das Wundabdeckmaterial-Kit nach einem der Ansprüche 17 bis 20, wobei das Wundabdeckmaterial mindestens ein Additiv, ausgewählt aus Blutgerinnungsfaktor XIII, einem Proteaseinhibitor, Calciumchlorid, Albumin, Natriumchlorid, Natriumcitrat oder einem nicht-ionischen Detergens, umfasst.

22. Das Wundabdeckmaterial-Kit nach Anspruch 21, wobei das Calciumchlorid dem bioabsorbierbaren Trägermaterial, das Thrombin enthält, als ein Additiv für Thrombin zugegeben ist.

23. Das Wundabdeckmaterial-Kit nach Anspruch 21, wobei das nicht-ionische Detergens in dem bioabsorbierbaren Trägermaterial, das Fibrinogen enthält, enthalten ist.

24. Das Wundabdeckmaterial-Kit nach einem der Ansprüche 17 bis 23, wobei das bioabsorbierbare Trägermaterial, das Thrombin enthält, oder das bioabsorbierbare Trägermaterial, das Fibrinogen enthält, mit Hilfe eines Verfahrens hergestellt ist, das den Schritt des Eintauchens eines bioabsorbierbaren Trägermaterials in eine Thrombin oder Fibrinogen enthaltende Lösung und den Schritt des Lyophilisierens des durch diesen Schritt erhaltenen Trägermaterials umfasst.

25. Das Wundabdeckmaterial-Kit nach einem der Ansprüche 17 bis 24, wobei das Wundabdeckmaterial eine schadhafte Stelle in der Haut aufgrund einer Verletzung, eines chirurgischen Eingriffs oder einer Verbrennung reparieren kann.

26. Ein Kit für das Wundabdeckmaterial wie in Anspruch 1 definiert, wobei das Kit einen Behälter, der Thrombin als einen wirksamen Bestandteil enthält, einen Behälter, der Fibrinogen als einen wirksamen Bestandteil enthält, ein bioabsorbierbares Trägermaterial und ein Abdeckmaterial zum Rückhalt von Feuchtigkeit, das aus Silikon oder Polyurethan besteht, umfasst.

27. Das Wundabdeckmaterial-Kit nach Anspruch 26, wobei das bioabsorbierbare Trägermaterial aus einem Material, ausgewählt aus der Gruppe bestehend aus Polyglycolsäure, Polymilchsäure und einem Copolymer von Glycolsäure und Milchsäure, besteht.

28. Das Wundabdeckmaterial-Kit nach Anspruch 26 oder 27, wobei das bioabsorbierbare Trägermaterial zu einem Vlies verarbeitet ist.

29. Das Wundabdeckmaterial-Kit nach einem der Ansprüche 26 bis 28, wobei das bioabsorbierbare Trägermaterial ein Vlies aus einem Polyglycolsäure-Material ist.

30. Das Wundabdeckmaterial-Kit nach einem der Ansprüche 26 bis 29, wobei das Wundabdeckmaterial mindestens ein Additiv, ausgewählt aus Blutgerinnungsfaktor XIII, einem Proteaseinhibitor, Calciumchlorid, Albumin, Natriumchlorid, Natriumcitrat oder einem nicht-ionischen Detergens, umfasst.

31. Das Wundabdeckmaterial-Kit nach Anspruch 30, wobei das Calciumchlorid dem Thrombin enthaltenden Behälter als ein Additiv für Thrombin zugegeben ist.

32. Das Wundabdeckmaterial-Kit nach Anspruch 30, wobei der Blutgerinnungsfaktor XIII in dem Fibrinogen enthaltenden Behälter enthalten ist.

33. Das Wundabdeckmaterial-Kit nach einem der Ansprüche 26 bis 32, wobei das Wundabdeckmaterial eine schadhafte Stelle in der Haut aufgrund einer Verletzung, eines chirurgischen Eingriffs oder einer Verbrennung reparieren kann.

## Revendications

1. Matériau de recouvrement de plaie comprenant une première couche comprenant de la thrombine et du fibrinogène à titre de substances efficaces et un matériau de support bioabsorbable à titre de substrat, ladite couche étant recouverte d'une seconde couche comprenant un matériau de couverture pour retenir l'humidité qui est fait de silicone ou de polyuréthane.

2. Matériau de recouvrement de plaie selon la revendication 1, dans lequel ladite première couche consiste soit en
(1) du fibrinogène et un matériau de support bioabsorbable renfermant la thrombine ; soit
(2) un matériau de support bioabsorbable renfermant la thrombine, et un matériau de support bioabsorbable renfermant le fibrinogène ; ou
(3) de la thrombine, du fibrinogène et un matériau de support bioabsorbable ;
dans lequel la thrombine et le fibrinogène sont séparés l'un de l'autre immédiatement avant utilisation.

3. Matériau de recouvrement de plaie selon la revendication 1 ou 2, dans lequel ledit matériau de support bioabsorbable est fait d'un matériau choisi dans le groupe constitué par l'acide polyglycolique, l'acide polylactique et un copolymère d'acide glycolique et d'acide lactique.

4. Matériau de recouvrement de plaie selon l'une quelconque des revendications 1 à 3, dans lequel ledit matériau de support bioabsorbable est converti en tissu non tissé.

5. Matériau de recouvrement de plaie selon l'une quelconque des revendications 1 à 4, dans lequel ledit matériau de support bioabsorbable est un tissu non tissé constitué d'un matériau fait d'acide polyglycolique.

6. Matériau de recouvrement de plaie selon l'une quelconque des revendications 1 à 5, dans lequel ledit matériau de recouvrement de plaie comprend au moins un additif choisi parmi le facteur XIII de coagulation sanguine, un inhibiteur de protéase, le chlorure de calcium, l'albumine, le chlorure de sodium, le citrate de sodium, ou un détergent non ionique.

7. Matériau de recouvrement de plaie selon l'une quelconque des revendications 1 à 6, dans lequel ledit matériau de recouvrement de plaie peut réparer une zone déficiente de la peau due à un traumatisme, une opération chirurgicale ou une brûlure.

8. Kit pour matériau de recouvrement de plaie tel que défini dans la revendication 1, ledit kit comprenant un matériau de support bioabsorbable renfermant la thrombine à titre de substance efficace, un récipient contenant le fibrinogène à titre de substance efficace, et un matériau de couverture pour retenir l'humidité fait de silicone ou de polyuréthane.

9. Kit pour matériau de recouvrement de plaie selon la revendication 8, dans lequel ledit matériau de support bioabsorbable est fait d'un matériau choisi dans le groupe constitué par l'acide polyglycolique, l'acide polylactique et un copolymère d'acide glycolique et d'acide lactique.

10. Kit pour matériau de recouvrement de plaie selon la revendication 8 ou 9, dans lequel ledit matériau de support bioabsorbable est converti en tissu non tissé.

11. Kit pour matériau de recouvrement de plaie selon l'une quelconque des revendications 8 à 10, dans lequel ledit matériau de support bioabsorbable est un tissu non tissé constitué d'un matériau d'acide polyglycolique.

12. Kit pour matériau de recouvrement de plaie selon l'une quelconque des revendications 8 à 11, dans lequel ledit matériau de recouvrement de plaie comprend au moins un additif choisi parmi le facteur XIII de coagulation sanguine, un inhibiteur de protéase, le chlorure de calcium, l'albumine, le chlorure de sodium, le citrate de sodium, ou un détergent non ionique.

13. Kit pour matériau de recouvrement de plaie selon la revendication 12, dans lequel ledit chlorure de calcium est ajouté au matériau de support bioabsorbable renfermant la thrombine à titre d'additif pour thrombine.

14. Kit pour matériau de recouvrement de plaie selon la revendication 12, dans lequel ledit facteur XIII de coagulation sanguine est contenu dans le récipient contenant le fibrinogène.

15. Kit pour matériau de recouvrement de plaie selon l'une quelconque des revendications 8 à 14, dans lequel ledit matériau de support bioabsorbable est préparé par un procédé comprenant l'étape d'immersion d'un matériau de support bioabsorbable dans une solution contenant la thrombine, et l'étape de lyophilisation du matériau de support obtenu par ladite étape.

16. Kit pour matériau de recouvrement de plaie selon l'une quelconque des revendications 8 à 15, dans lequel ledit matériau de recouvrement de plaie peut réparer une zone déficiente de la peau due à un traumatisme, une opération chirurgicale ou une brûlure.

17. Kit pour matériau de recouvrement de plaie tel que défini dans la revendication 1, ledit kit comprenant un matériau de support bioabsorbable renfermant la thrombine à titre de substance efficace, un matériau de support bioabsorbable renfermant le fibrinogène à titre de substance efficace, et un matériau de couverture pour retenir l'humidité fait de silicone ou polyuréthane.

18. Kit pour matériau de recouvrement de plaie selon la revendication 17, dans lequel ledit matériau de support bioabsorbable est fait d'un matériau choisi dans le groupe constitué par l'acide polyglycolique, l'acide polylactique et un copolymère d'acide glycolique et d'acide lactique.

19. Kit pour matériau de recouvrement de plaie selon la revendication 17 ou 18, dans lequel ledit matériau de support bioabsorbable est converti en tissu non tissé.

20. Kit pour matériau de recouvrement de plaie selon l'une quelconque des revendications 17 à 19, dans lequel ledit matériau de support bioabsorbable est un tissu non tissé constitué d'un matériau d'acide polyglycolique.

21. Kit pour matériau de recouvrement de plaie selon l'une quelconque des revendications 17 à 20, dans lequel ledit matériau de recouvrement de plaie comprend au moins un additif choisi parmi le facteur XIII de coagulation sanguine, un inhibiteur de protéase, le chlorure de calcium, l'albumine, le chlorure de sodium, le citrate de sodium, ou un détergent non ionique.

22. Kit pour matériau de recouvrement de plaie selon la revendication 21, dans lequel ledit chlorure de calcium est ajouté au matériau de support bioabsorbable renfermant la thrombine à titre d'additif pour thrombine.

23. Kit pour matériau de recouvrement de plaie selon la revendication 21, dans lequel ledit détergent non ionique est contenu dans le matériau de support bioabsorbable renfermant le fibrinogène.

24. Kit pour matériau de recouvrement de plaie selon l'une quelconque des revendications 17 à 23, dans lequel ledit matériau de support bioabsorbable renfermant la thrombine ou ledit matériau de support bioabsorbable renfermant le fibrinogène est préparé par un procédé comprenant l'étape d'immersion d'un matériau de support bioabsorbable dans une solution contenant la thrombine ou le fibrinogène, et l'étape de lyophilisation du matériau de support obtenu par ladite étape.

25. Kit pour matériau de recouvrement de plaie selon l'une quelconque des revendications 17 à 24, dans lequel ledit matériau de recouvrement de plaie peut réparer une zone déficiente de la peau due à un traumatisme, une opération chirurgicale ou une brûlure.

26. Kit pour matériau de recouvrement de plaie tel que défini dans la revendication 1, ledit kit comprenant un récipient contenant la thrombine à titre de substance efficace, un récipient contenant le fibrinogène à titre de substance efficace, un matériau de support bioabsorbable, et un matériau de couverture pour retenir l'humidité qui est fait de silicone ou polyuréthane.

27. Kit pour matériau de recouvrement de plaie selon la revendication 26, dans lequel ledit matériau de support bioabsorbable est fait d'un matériau choisi dans le groupe constitué par l'acide polyglycolique, l'acide polylactique et un copolymère d'acide glycolique et d'acide lactique.

28. Kit pour matériau de recouvrement de plaie selon la revendication 26 ou 27, dans lequel ledit matériau de support bioabsorbable est converti en tissu non tissé.

29. Kit pour matériau de recouvrement de plaie selon l'une quelconque des revendications 26 à 28, dans lequel ledit matériau de support bioabsorbable est un tissu non tissé constitué d'un matériau d'acide polyglycolique.

30. Kit pour matériau de recouvrement de plaie selon l'une quelconque des revendications 26 à 29, dans lequel ledit matériau de recouvrement de plaie comprend au moins un additif choisi parmi le facteur XIII de coagulation sanguine, un inhibiteur de protéase, le chlorure de calcium, l'albumine, le chlorure de sodium, le citrate de sodium, ou un détergent non ionique.

31. Kit pour matériau de recouvrement de plaie selon la revendication 30, dans lequel ledit chlorure de calcium est ajouté au récipient contenant la thrombine à titre d'additif pour thrombine.

32. Kit pour matériau de recouvrement de plaie selon la revendication 30, dans lequel ledit facteur XIII de coagulation sanguine est contenu dans le récipient contenant le fibrinogène.

33. Kit pour matériau de recouvrement de plaie selon l'une quelconque des revendications 26 à 32, dans lequel ledit matériau de recouvrement de plaie peut réparer une zone déficiente de la peau due à un traumatisme, une opération chirurgicale ou une brûlure.
